# EUROPEAN PATENT SPECIFICATION

(11) **EP 3 128 938 B1**
(45) Date of publication and mention of the grant of the patent: **27.06.2018**
(21) Application number: 15734245.2
(22) Date of filing: 26.03.2015
(51) Int. Cl.: A61B 18/14

(54) **BIPOLAR DEVICE FOR THE SURGICAL RESECTION OF TISSUES**
BIPOLARE VORRICHTUNG ZUR CHIRURGISCHEN RESEKTION VON GEWEBEN
DISPOSITIF BIPOLAIRE POUR LA RÉSECTION CHIRURGICALE DE TISSUS

(30) Priority: 09.04.2014 IT MO20140099
(43) Date of publication of application: 15.02.2017
(73) Proprietor: BTC Medical Europe S.R.L., 37067 Valeggio sul Mincio (VR) (IT)
(72) Inventor: BOZZA, Marco, 37067 Valeggio sul Mincio (VR) (IT)
(74) Representative: Zoli, Filippo
(86) International application number: PCT/IT2015/000082
(87) International publication number: WO 2015/155794

(56) References cited:
- DE-A1- 2 514 501
- GB-A- 2 485 569
- US-A- 5 078 716
- US-A1- 2009 182 324

## Description

### Technical Field

The present invention relates to a bipolar device for the surgical resection of tissues.

### Background Art

Currently known surgical techniques often envisage the use of so-called electrosurgical units, i.e., devices suitable for applying an electric discharge on the patient's body, the thermal effect of which causes a transformation of the cells of the tissue involved which can vary depending on the temperature reached, leading to the resection or cauterization of the body tissue.

These devices of known type, which can be either unipolar or bipolar, generally have two electrodes, between which the electric discharge occurs and between which the tissue to be re-sectioned is placed.

The present invention relates particularly to devices for the surgical resection of tissues of the bipolar type.

In particular, devices are known that envisage two electrically conductive filaments, which define the two electrodes mentioned above and which are associated with one another to define a loop inside which the portion of tissue to be re-sectioned is inserted. These filaments are inserted sliding inside a tubular sheath in such a way as to produce the opening and closing of the loop itself.

Two known devices are described by US 6050995 and US 4493320.

More in detail, US 6050995 describes a device in which the two electrodes are intertwined with one another to define the two above-mentioned filaments.

This device therefore leads to the formation of a plurality of voltaic arcs of reduced dimensions along each filament.

The device described by US 6050995 is of limited effectiveness due to the reduced dimensions of the photovoltaic arcs which are defined along each filament.

The device described by US 4493320 on the other hand envisages that both the electrically conductive filaments be uncovered and that the tubular sheath be of the double-channel type, inside each of which channels a relative filament is inserted.

This device also has drawbacks because the presence of the double-channel tubular sheath, and more precisely of the wall that separates the channels themselves the one from the other, does not permit the correct and complete closing of the loop defined by the two filaments and therefore obtaining a precise resection.

What is more, the fact that the filaments are uncovered results in high energy dispersion, as well as the risk of a short circuit following the closing of the loop as a result of the contact between the filaments themselves. Document GB-A-2 485 569 discloses the most relevant prior art.

### Description of the Invention

The main aim of the present invention is to provide a bipolar device for the surgical resection of tissues which operates efficiently and accurately.

Within this aim, one object of the present invention is to provide a device which allows the complete closure of the loop defined by the respective filaments and which, at the same time, prevents a short circuit from occurring.

Another object of the present invention is to provide a bipolar device for the surgical resection of tissues which allows to overcome the mentioned drawbacks of the prior art within the ambit of a simple, rational, easy and effective to use as well as affordable solution.

The above mentioned objects are achieved by the present bipolar device for the surgical resection of tissues according to claim 1.

### Brief Description of the Drawings

Other characteristics and advantages of the present invention will become more evident from the description of a preferred, but not exclusive embodiment of a bipolar device for the surgical resection of tissues, illustrated by way of an indicative, but not limitative example in the accompanying drawings in which:
Figure 1 is a side elevational view of a device according to the invention;
Figure 2 is an enlargement of a detail of the device of Figure 1.

### Embodiments of the Invention

With particular reference to these illustrations, generally indicated by reference number 1 is a bipolar device for the surgical resection of tissues.

The device 1 comprises two insulated and electrically conductive filaments 2, 3, each of which has a relative proximal extremity 2a, 3a and a relative distal extremity 2b, 3b.

More in detail, the device 1 comprises a first filament 2 and a second filament 3, the distal extremities of which, 2b and 3b respectively, are connected together by means of insulating means 4 to define a loop 5 intended to accommodate the portion of tissue to be re-sectioned.

Preferably, the first and the second filament 2 and 3 are of the flexible and monofilament type.

The insulating means 4 are made e.g. of a material comprising a ceramic-based insulating resin.

The device 1 then comprises at least a tubular element 6 inside which the filaments 2 and 3 are inserted sliding.

More in particular, the tubular element 6 is of the single channel type and the filaments 2, 3 have a greater longitudinal extension than that of the tubular element itself. The filaments 2 and 3 therefore protrude from both the opposite longitudinal extremities of the tubular element 6.

By effect of the sliding of the filaments 2 and 3 with respect to the tubular element 6, the loop 5 opens and closes depending on the sliding direction. In particular, the loop 5 behaves elastically and consequently tends to open automatically as the distal extremities 2b, 3b of the filaments 2, 3 gradually come out of the tubular element 6.

Suitably, the tubular element 6 is made of Teflon.

The device 1 also comprises connection means 7 for electrically connecting the filaments 2 and 3 to a bipolar current source (not shown in the illustrations). Advantageously, the device 1 comprises activation means 8 of the sliding of the filaments 2, 3 with respect to the tubular element 6.

More in detail, the activation means 8 comprise a body 9, of the rigid type, inside which the tubular element 6 is at least partially fitted and which has a grip 10, and comprise at least an activation element 11, which is mobile sliding with respect to the body 9 and which is associated integral with a section of the filaments 2, 3.

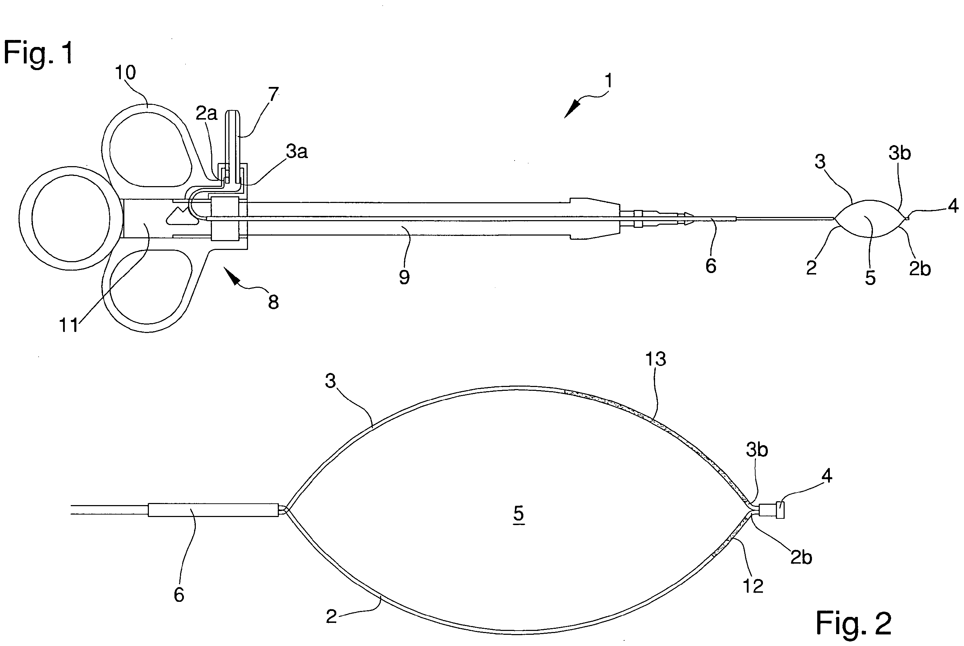

As shown in figure 1, the body 9 also supports the connection means 7 for electrically connecting the filaments 2 and 3 to the bipolar current source. More in particular, the connection means 7 comprise a connector made of electrically conductive material, e.g., stainless steel, which has a portion arranged inside the body 9, to which are connected the proximal extremities 2a, 3a of the filaments 2 and 3, and a portion that protrudes outside the body itself, which can be connected to the bipolar current source.

The activation element 11 is therefore associated with the portion of the filaments 2, 3 placed between their proximal extremities 2a, 3a and the closest longitudinal extremity of the tubular element 6. By operating the activation element 11 the sliding is therefore caused of the filaments 2, 3 and, consequently, the opening/closing of the loop 5.

Each of the filaments 2 and 3 has a relative uncovered section, identified by the reference numbers 12 and 13 respectively.

More in particular, along the first filament 2 a first uncovered section 12 is defined and along the second filament 3 a second uncovered section 13 is defined.

Such uncovered sections 12 and 13 have different extensions the one from the other and are suitable for acting as a positive electrode and a negative electrode respectively, in such a way that between them an electric discharge occurs for the resection of the tissue inserted inside the loop 5 and therefore placed between the electrodes themselves. The tissue inserted through the loop 5 therefore acts as an electric bridge, allowing the current to pass from one electrode to another.

More in particular, the sections 12 and 13 are defined along the loop 5 and the current passes from the section 12, 13 having greater extension to the section 13, 12 having minor extension.

Preferably, the sections 12 and 13 are arranged at the respective distal extremities 2b and 3b.

Alternative embodiments cannot however be ruled out wherein the sections 12 and 13 are positioned differently along the loop 5.

According to the invention, the first section 12 has an extension between 3mm and 8mm, preferably 5mm, and the second section 13 has an extension between 15mm and 25mm, preferably 20mm.

Alternative embodiments cannot however be ruled out wherein the sections 12, 13 have extensions different to those mentioned above.

The operation of the present invention is the following.

The operator initially intervenes on the activation means 8 to move the distal extremities 2b, 3b away from the tubular element 6, so that the loop 5 moves to its maximum opening configuration.

At this point, the operator moves the device 1 so as to insert the portion of tissue to undergo resection inside the loop 5.

Subsequently, intervention is again made on the activation means 8, this time to move the distal extremities 2b, 3b near to the tubular element 6, in such a way as to tighten the loop 5.

This way, the two portions of the filaments 2 and 3 which define the loop 5 move nearer to one another until they come into contact with the tissue to undergo resection.

The moment the uncovered sections 12 and 13 touch the tissue to undergo resection, an electric discharge occurs between them that crosses the tissue itself.

By further closing the loop 5, until it substantially fully returns again inside the tubular element 6, the cut is obtained of the tissue inserted inside the loop itself. It has in practice been ascertained how the described invention achieves the proposed objects and in particular the fact is underlined that the presence of two uncovered sections, having a limited extension and different the one to the other, permits obtaining an electric discharge that concentrates between the uncovered sections themselves. This way, highly effective cut and reduced energy dispersion are achieved.

## Claims

1. Bipolar device (1) for the surgical resection of tissues, comprising:
- two insulated and electrically conductive filaments (2, 3), which have a relative proximal extremity (2a, 3a) and a relative distal extremity (2b, 3b), where said distal extremities (2b, 3b) are connected together by means of insulating means (4) to define a loop (5) intended to accommodate the portion of tissue to be re-sectioned, where each of said filaments (2, 3) has a relative uncovered section (12, 13), of which a first section (12) and a second section (13), having a limited extension and suitable for acting as a positive electrode and a negative electrode respectively, in such a way that between them an electric discharge occurs for the resection of the tissue inserted inside said loop (5) and placed between the electrodes themselves;
- at least a tubular element (6) inside which both said filaments (2, 3) are inserted sliding, said loop (5) being suitable for opening and closing by effect of the sliding of said filaments inside said tubular element (6);
- connection means (7) for electrically connecting said filaments to a bipolar current source;
**characterized by** the fact that said first section (12) has an extension between 3mm and 8mm and that said second section (13) has an extension between 15mm and 25mm.

2. Device (1) according to claim 1, **characterized by** the fact that said sections (12, 13) are defined along said loop (5).

3. Device (1) according to claim 2, **characterized by** the fact that said sections (12, 13) are arranged at the respective distal extremities (2b, 3b).

4. Device (1) according to one or more of the preceding claims, **characterized by** the fact that said insulating means (4) are made of a material comprising a ceramic-based resin.

5. Device (1) according to one or more of the preceding claims, **characterized by** the fact that it comprises activation means (8) of the sliding of said filaments (2, 3) with respect to said tubular element (6).

6. Device (1) according to one or more of the preceding claims, **characterized by** the fact that said filaments (2, 3) are of the monofilament type.

## Patentansprüche

1. Bipolare Vorrichtung (1) für die chirurgische Resektion von Geweben, umfassend:
- zwei isolierte und elektrisch leitende Filamente (2, 3), die einen relativen proximalen Endpunkt (2a, 3a) und einen relativen distalen Endpunkt (2b, 3b) aufweisen, wobei die distalen Endpunkte (2b, 3b) miteinander verbunden sind mittels Isolierungsmitteln (4), um eine Schleife (5) zu definieren, die dafür vorgesehen ist, den Abschnitt des Gewebes aufzunehmen, der resektioniert wird, wobei jedes der Filamente (2, 3) einen relativ unbedeckten Abschnitt (12, 13) aufweist, wobei ein erster Abschnitt (12) und ein zweiter Abschnitt (13) eine limitierte Ausdehnung haben und geeignet sind, als positive Elektrode beziehungsweise als negative Elektrode zu fungieren, derart, dass zwischen ihnen eine elektrische Entladung auftritt, zur Resektion des Gewebes, das in die Schleife (5) eingefügt wurde und zwischen den Elektroden selbst angeordnet wurde;
- mindestens ein rohrförmiges Element (6), innerhalb dessen beide Filamente (2, 3) gleitend eingefügt sind, wobei die Schleife (5) durch das Gleiten der Filamente innerhalb des rohrförmigen Elements (6) zum Öffnen und Schließen geeignet ist;
- Verbindungsmittel (7) zum elektrischen Anschließen der Filamente an eine zweipolige Stromquelle;
**dadurch gekennzeichnet, dass** der erste Abschnitt (12) eine Ausdehnung zwischen 3 mm und 8 mm aufweist und dass der zweite Abschnitt (13) eine Ausdehnung zwischen 15 mm und 25 mm aufweist.

2. Vorrichtung (1) nach Anspruch 1, **dadurch gekennzeichnet, dass** die Abschnitte (12, 13) entlang der Schleife (5) definiert sind.

3. Vorrichtung (1) nach Anspruch 2, **dadurch gekennzeichnet, dass** die Abschnitte (12, 13) an den relativen, distalen Endpunkten (2b, 3b) angeordnet sind.

4. Vorrichtung (1) nach einem oder mehreren der vorhergehenden Ansprüche, **dadurch gekennzeichnet, dass** die Isolierungsmittel (4) aus einem Material hergestellt sind, das ein auf Keramik basierendes Harz umfasst.

5. Vorrichtung (1) nach einem oder mehreren der vorhergehenden Ansprüche, **dadurch gekennzeichnet, dass** sie Aktivierungsmittel (8) des Gleitens der Filamente (2, 3) in Bezug auf das rohrförmige Element (6) umfasst.

6. Vorrichtung (1) nach einem oder mehreren der vorhergehenden Ansprüche, **dadurch gekennzeichnet, dass** die Filamente (2, 3) vom Typ der Monofilamente sind.

## Revendications

1. Dispositif bipolaire (1) pour la résection chirurgicale de tissus, comprenant :
- deux filaments électroconducteurs et isolés (2, 3), qui ont une extrémité proximale relative (2a, 3a) et une extrémité distale relative (2b, 3b), dans lequel lesdites extrémités distales (2b, 3b) sont raccordées l'une à l'autre par un moyen d'isolation (4) pour définir une boucle (5) destinée à recevoir la partie de tissu à réséquer, dans lequel chacun des filaments (2, 3) comporte une section non couverte relative (12, 13), dont une première section (12) et une seconde section (13), présentant une extension limitée et appropriée pour agir en tant qu'électrode positive et électrode négative respectivement, afin qu'il se produise entre elles une décharge électrique pour permettre la résection du tissu inséré à l'intérieur de ladite boucle (5) et placé entre les électrodes proprement dites ;
- au moins un élément tubulaire (6) à l'intérieur duquel les deux filaments (2, 3) sont introduits de manière coulissante, ladite boucle (5) étant appropriée pour s'ouvrir et se fermer sous l'effet du coulissement desdits filaments à l'intérieur de l'élément tubulaire (6) ;
- un moyen de connexion (7) pour connecter électriquement lesdits filaments à une source de courant bipolaire ;
**caractérisé par le fait que** ladite première section (12) comporte une extension entre 3 mm et 8 mm et ladite seconde section (13) comporte une extension entre 15 mm et 25 mm.

2. Dispositif (1) selon la revendication 1, **caractérisé par le fait que** lesdites sections (12, 13) sont définies le long de ladite boucle (5).

3. Dispositif (1) selon la revendication 2, **caractérisé par le fait que** lesdites sections (12, 13) sont agencées au niveau des extrémités distales respectives (2b, 3b).

4. Dispositif (1) selon une ou plusieurs des revendications précédentes, **caractérisé par le fait que** ledit moyen d'isolation (4) est en un matériau comprenant une résine à base de céramique.

5. Dispositif (1) selon une ou plusieurs des revendications précédentes, **caractérisé par le fait qu'**il comprend un moyen d'activation (8) du coulissement desdits filaments (2, 3) relativement audit élément tubulaire (6).

6. Dispositif (1) selon une ou plusieurs des revendications précédentes, **caractérisé par le fait que** lesdits filaments (2, 3) sont du type monofilament.
